# EUROPEAN PATENT APPLICATION

(11) **EP 4 772 216 A1**
(43) Date of publication of application: **08.07.2026**
(21) Application number: 25181738.3
(22) Date of filing: 10.06.2025
(51) Int. Cl.: A61M 5/32, A61J 1/20, A61M 5/20, A61M 5/31

(54) **INJECTION DEVICE**

(30) Priority: 07.01.2025 US 202563742849 P; 27.05.2025 US 202519220028
(71) Applicant: Altek Biotechnology Corporation, 300 Hsinchu City (TW)
(72) Inventor: CHU, Yi, 300 Hsinchu City (TW); YAO, Jyun-An, 300 Hsinchu City (TW)
(74) Representative: Straus, Alexander

(57) **Abstract**

An injection device(1A,1B,1C,1D) is provided and includes an injector body(11A,11B), a reservoir(12A), a needle hub(13A,13C), a cap(14A,14C), a vial adaptor(15A,15C) and a drug needle(16A,16B,16C,16D,16). The reservoir(12A) is disposed on the injector body(11A,11B). The needle hub(13A,13C) is engaged with the reservoir(12A). The cap(14A,14C) is removably mounted on the injector body(11A,11B). The vial adaptor(15A,15C) is separably engaged with the needle hub(13A,13C). The vial adaptor(15A,15C) is configured to hold a vial member(2A,2C,2D). The drug needle(16A,16B,16C,16D,16) is disposed on the needle hub(13A,13C) and configured to provide a drug passage communicated with an interior of the reservoir(12A) for allowing drug to flow from an interior of the vial member(2A,2C,2D) to the interior of the reservoir(12A).

## Description

### Field of the Invention

The present invention relates to an injection device according to the pre-characterizing clause of claim 1.

### Background of the Invention

An injection device is designed for drug delivery. The conventional injection devices are typically configured to accommodate pre-filled drug cartridges, which are directly inserted into the injection devices. However, in many applications, medications are commonly supplied in vial form. Such injection devices do not have any transferring mechanism for transferring drug from a vial member to a drug reservoir and therefore are not suitable for vial-based drug preparation. Accordingly, in order to meet different requirements, an improvement of the injection device is urgently needed.

### Summary of the Invention

This is mind, the present invention aims at providing an injection device with enhanced reliability and operational convenience in drug administration.

This is achieved by an injection device according to claim 1. The dependent claims pertain to corresponding further developments and improvements.

As will be seen more clearly from the detail description following below, the claimed injection device includes an injector body, a reservoir, a needle hub, a cap, a vial adaptor and a drug needle. The reservoir is disposed on the injector body. The needle hub is engaged with the reservoir. The cap is removably mounted on the injector body. The vial adaptor is separably engaged with the needle hub. The vial adaptor is configured to hold a vial member. The drug needle is disposed on the needle hub and configured to provide a drug passage communicated with an interior of the reservoir for allowing drug to flow from an interior of the vial member to the interior of the reservoir.

In summary, the injection device of the present invention is suitable for vial-based drug preparation and capable of preventing the drug needle from being deformed or damaged due to a torsional force during removal of the vial member. As a result, the injection device of the present invention enhances reliability and operational convenience in drug administration.

These and other objectives of the present invention will no doubt become obvious to those of ordinary skill in the art after reading the following detailed description of the preferred embodiment that is illustrated in the various figures and drawings.

### Brief Description of the Drawings

In the following, the invention is further illustrated by way of example, taking reference to the accompanying drawings. Thereof:
FIG. 1 is a schematic diagram of an injection device according to a first embodiment of the present invention,
FIG. 2 and FIG. 3 are exploded diagrams of the injection device at different views according to the first embodiment of the present invention,
FIG. 4 is a partial sectional diagram of the injection device according to the first embodiment of the present invention,
FIG. 5 and FIG. 6 are partial diagrams of the injection device at different views according to the first embodiment of the present invention,
FIG. 7 is a partial diagram of a cap according to the first embodiment of the present invention,
FIG. 8 to FIG. 10 are partial diagrams of the injection device in different states according to the first embodiment of the present invention,
FIG. 11 to FIG. 13 are partial diagrams of the injection device according to different embodiments of the present invention,
FIG. 14 is a partial diagram of an injection device according to a second embodiment of the present invention,
FIG. 15 is a partial exploded diagram of the injection device according to the second embodiment of the present invention,
FIG. 16 to FIG. 20 are partial diagrams of the injection device in different states according to the second embodiment of the present invention,
FIG. 21 is a partial exploded diagram of an injection device according to a third embodiment of the present invention,
FIG. 22 is a partial sectional diagram of the injection device according to the third embodiment of the present invention,
FIG. 23 is a partial diagram of a cap according to the third embodiment of the present invention,
FIG. 24 is a partial exploded diagram of an injection device according to a fourth embodiment of the present invention, and
FIG. 25 is a partial sectional diagram of the injection device according to the fourth embodiment of the present invention.

### Detailed Description

In the following detailed description of the preferred embodiments, reference is made to the accompanying drawings which form a part hereof, and in which is shown by way of illustration specific embodiments in which the invention may be practiced. In this regard, directional terminology, such as "top", "bottom", "left", "right", "front", "back", etc., is used with reference to the orientation of the Figure(s) being described. The members of the present invention can be positioned in a number of different orientations. As such, the directional terminology is used for purposes of illustration and is in no way limiting. Accordingly, the drawings and descriptions will be regarded as illustrative in nature and not as restrictive. Also, if not specified, the term "connect" is intended to mean either an indirect or direct mechanical connection. Thus, if a first device is connected to a second device, that connection may be through a direct mechanical connection, or through an indirect mechanical connection via other devices and connections.

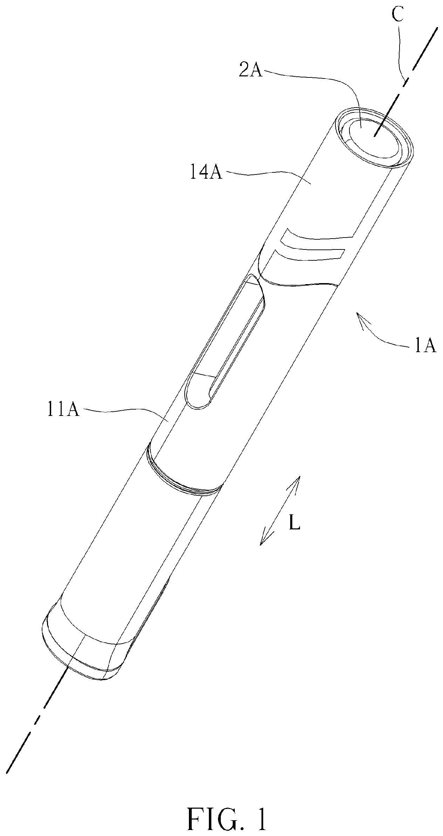

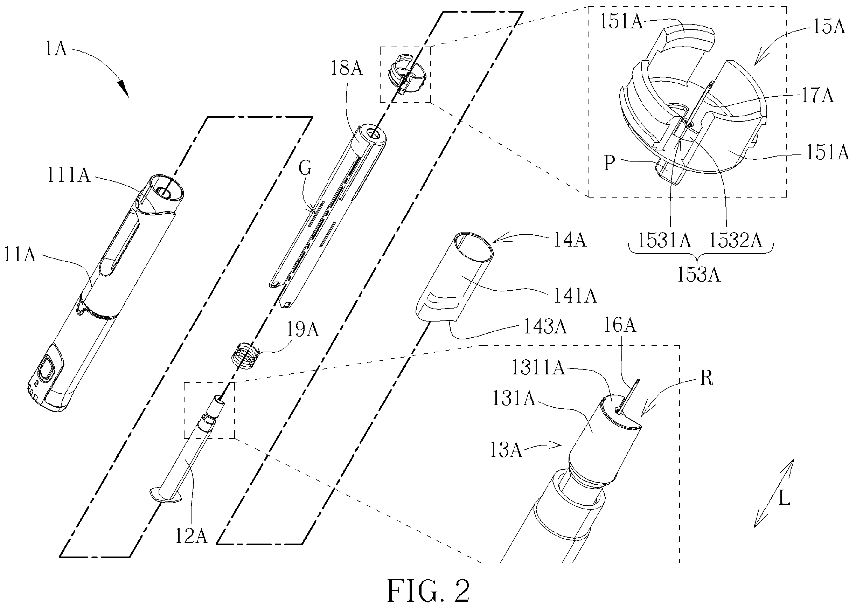

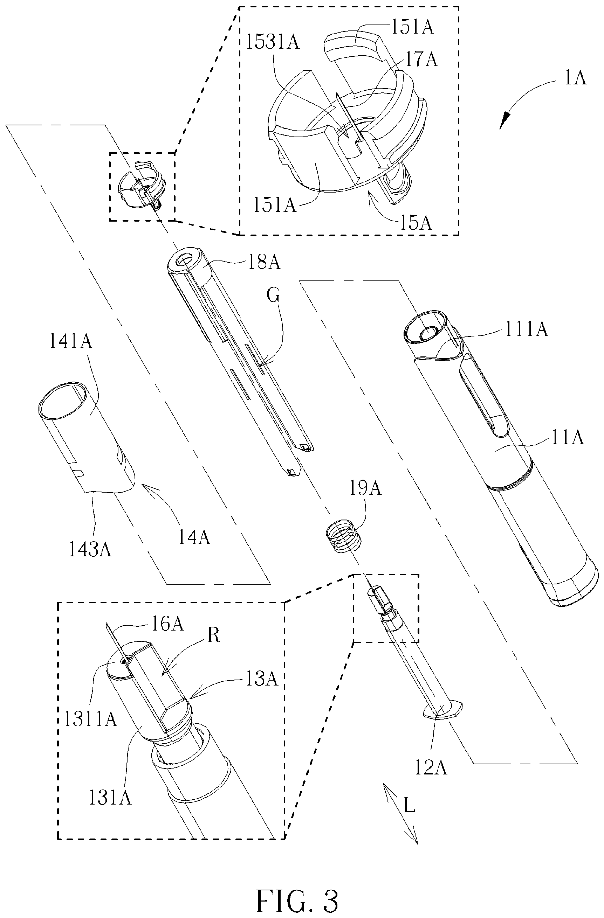

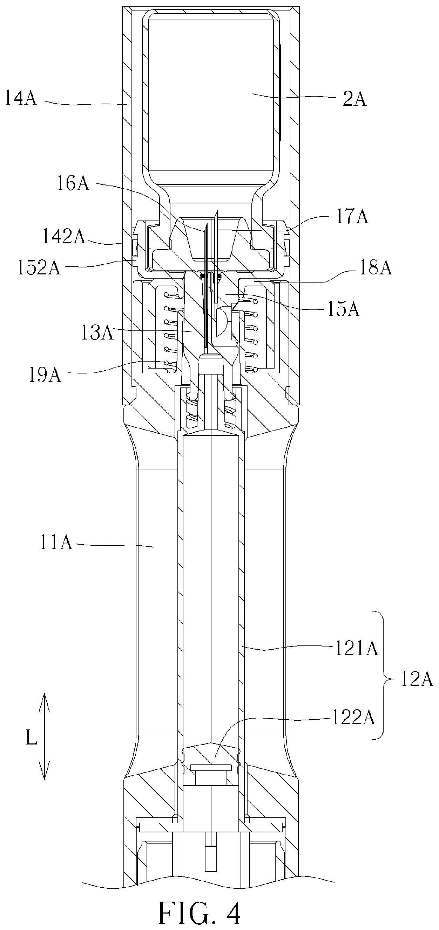

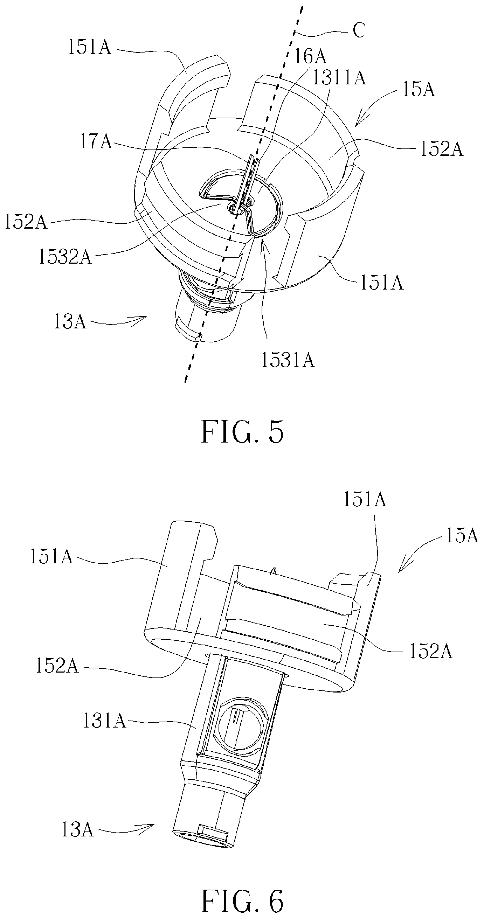

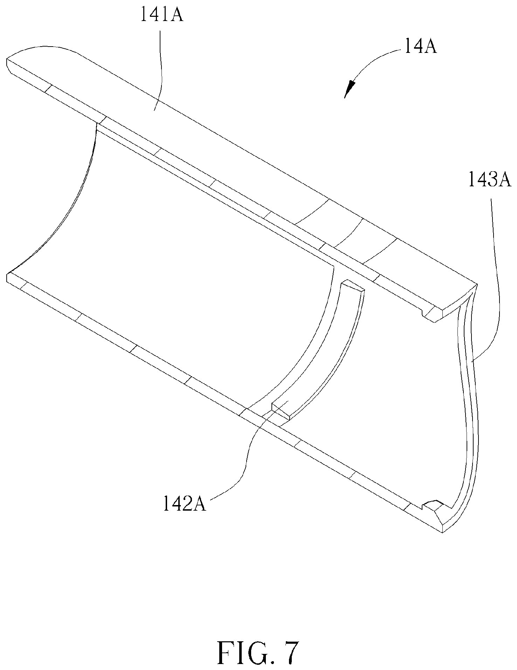

Please refer to FIG. 1 to FIG. 7. FIG. 1 is a schematic diagram of an injection device 1A according to a first embodiment of the present invention. FIG. 2 and FIG. 3 are exploded diagrams of the injection device 1A at different views according to the first embodiment of the present invention. FIG. 4 is a partial sectional diagram of the injection device 1A according to the first embodiment of the present invention. FIG. 5 and FIG. 6 are partial diagrams of the injection device 1A at different views according to the first embodiment of the present invention. FIG. 7 is a partial diagram of a cap 14A according to the first embodiment of the present invention. As shown in FIG. 1 to FIG. 7, in the first embodiment, the injection device 1A is adapted for a vial member 2A and includes an injector body 11A, a reservoir 12A, a needle hub 13A, the cap 14A, a vial adaptor 15A and a drug needle 16A. The reservoir 12A is disposed on the injector body 11A. The needle hub 13A is engaged with the reservoir 12A. The cap 14A is removably mounted on the injector body 11A. The cap 14A is configured to be rotatable around a central axis C of the vial member 2A extending along a longitudinal direction L relative to the injector body 11A and movable along the longitudinal direction L relative to the injector body 11A. In this embodiment, the injection device 1A can be formed in a pen shape, and the longitudinal direction L is a length direction of the injector body 11A and/or a length direction of the injection device 1A. The vial adaptor 15A is separably engaged with the needle hub 13A. The vial adaptor 15A is configured to hold the vial member 2A. The drug needle 16A is disposed on the needle hub 13A and configured to provide a drug passage communicated with an interior of the reservoir 12A for allowing drug to flow from an interior of the vial member 2A to the interior of the reservoir 12A. For example, as shown in FIG. 4, the reservoir 12A can be a syringe having a barrel 121A received inside the injector body 11A and a plunger 122A movable relative to the barrel 121A, and the plunger 122A can be pulled along the longitudinal direction L manually or by an electrical motor to introduce the drug to flow from the interior of the vial member 2A to the interior of the reservoir 12A for drug transfer from the vial member 2A to the reservoir 12A.

Besides, the injection device 1A further includes an air needle 17A disposed on the vial adaptor 15A and configured to pierce into the vial member 2A to provide an air passage communicated with the interior of the vial member 2A for allowing air to flow to the interior of the vial member 2A, which achieves pressure balance for facilitating the drug transfer from the vial member 2A into the reservoir 12A. In this embodiment, both the drug needle 16A and the air needle 17A can be made of metal material. However, the present invention is not limited to this embodiment. For example, in another embodiment, the air needle can be made of plastic material, and the air needle can be integrally formed with the vial adaptor, e.g., by injection molding.

Preferably, the cap 14A is configured to receive the vial adaptor 15A and the vial member 2A, and the vial adaptor 15A is configured to receive a portion of the vial member 2A.

Specifically, as shown in FIG. 2 to FIG. 6, the vial adaptor 15A includes at least one resilient hook structure 151A configured to hold the vial member 2A. The cap 14A includes a wall section 141A configured to orientate the vial member 2A for aligning a length direction of the vial member 2A with the longitudinal direction L, and the wall section 141A encircles the vial member 2A when the resilient hook structure 151A holds the vial member 2A.

Preferably, as shown in FIG. 2 to FIG. 7, the cap 14A further includes at least one first rotating cooperating structure 142A (shown in FIG. 4 and FIG. 7), and the vial adaptor 15A further includes at least one second rotating cooperating structure 152A configured to rotatably engage with the first rotating cooperating structure 142A.

In this embodiment, the first rotating cooperating structure 142A and the second rotating cooperating structure 152A are a single-rib structure and a double-rib structure, respectively, and the single-rib structure can be movably located between two rib portions of the double-rib structure when the first rotating cooperating structure 142A and the second rotating cooperating structure 152A rotatably engage with each other. However, the present invention is not limited to this embodiment. For example, in another embodiment, the first rotating cooperating structure and the second rotating cooperating structure can be a double-rib structure and a single-rib structure which can be located between two rib portions of the double-rib structure, respectively.

Preferably, the first rotating cooperating structure 142A and the second rotating cooperating structure 152A are parallel to each other.

Preferably, the first rotating cooperating structure 142A extends along a circumferential direction of the cap 14A, and the second rotating cooperating structure 152A extends along a circumferential direction of the vial adaptor 15A.

When the cap 14A rotates relative to the injector body 11A around the central axis C of the vial member 2A extending along the longitudinal direction L, a rotating engagement of the first rotating cooperating structure 142A and the second rotating cooperating structure 152A enables the cap 14A to rotate relative to the vial adaptor 15A, without introducing rotation of the vial adaptor 15A, thereby preventing the drug needle 16A from being deformed or damaged due to a torsional force resulting from rotation of the vial member 2A relative to the drug needle 16A and/or the air needle 17A, which may be introduced by the rotation of the vial adaptor 15A relative to the needle hub 13A. When the cap 14A is removed from the injector body 11A along the longitudinal direction L, the rotating engagement of the first rotating cooperating structure 142A and the second rotating cooperating structure 152A enables the cap 14A to drive the vial adaptor 15A and the vial member 2A to move together with the cap 14A along the longitudinal direction L.

In this embodiment, the resilient hook structure 151A and the second rotating cooperating structure 152A are located at a same side of the vial adaptor 15A. However, the present invention is not limited to this embodiment. For example, in another embodiment, the resilient hook structure 151A and the second rotating cooperating structure 152A can located at two opposite sides of the vial adaptor 15A, respectively.

Furthermore, as shown in FIG. 2 and FIG. 3, in order for intuitive removal, the injector body 11A includes a first abutting structure 111A, and the cap 14A further includes a second abutting structure 143A configured to cooperate with the first abutting structure 111A to drive the cap 14A to move along the longitudinal direction L for removal of the cap 14A from the injector body 11A when the cap 14A rotates relative to the injector body 11A, thereby enabling a user to remove the cap 14A by applying a torsional force only, without a need for a longitudinal force.

In this embodiment, the first abutting structure 111A and the second abutting structure 143A are waved-surface structures. However, the present invention is not limited to this embodiment. For example, the first abutting structure 111A and the second abutting structure 143A can be curved-surface structures, inclined-surface structures, or threaded-surface structures.

Preferably, as shown in FIG. 2, FIG. 3, FIG. 5 and FIG. 6, the needle hub 13A includes a first rotating restraining structure 131A, and the vial adaptor 15A further includes a second rotating restraining structure 153A configured to non-rotatably engage with the first rotating restraining structure 131A when the vial adaptor 15A is engaged with the needle hub 13A. A non-rotating engagement of the first rotating restraining structure 131A and the second rotating restraining structure 153A restrains a relative rotating movement of the vial adaptor 15A and the needle hub 13A around the central axis C of the vial member 2A extending along the longitudinal direction L and allows a relative linear movement of the vial adaptor 15A and the needle hub 13A along the longitudinal direction L. The non-rotating engagement of the first rotating restraining structure 131A and the second rotating restraining structure 153A can effectively prevent any unintentional rotation of the vial adaptor 15A relative to the needle hub 13A, e.g., due to a frictional force that may be exerted between the first rotating cooperating structure 142A and the second rotating cooperating structure 152A during rotation of the cap 14A, thereby maintaining integrity of the drug needle 16A and/or the air needle 17A.

Specifically, the first rotating restraining structure 131A includes a first restraining feature 1311A, and the second rotating restraining structure 153A includes a restraining space 1531A and a second restraining feature 1532A. The first restraining feature 1311A is configured to stretch into the restraining space 1531A along the first restraining feature 1311A for the non-rotating engagement of the first rotating restraining structure 131A and the second rotating restraining structure 153A.

Preferably, a shape of a cross section of the restraining space 1531A and a shape of a cross section of the first restraining feature 1311A match with each other and are non-circular.

Preferably, a protruding section P is formed on the first restraining feature 1311A, and a recessed section R is formed on the second restraining feature 1532A and configured to at least partially receive the protruding section P.

Preferably, as show in FIG. 5, the air needle 17A is disposed on the second restraining feature 1532A of the second rotating restraining structure 153A of the vial adaptor 15A, and the drug needle 16A is disposed on the first restraining feature 1311A of the first rotating restraining structure 131A of the needle hub 13A.

In this embodiment, the air needle 17A and the drug needle 16A are located adjacent to each other, and the air needle 17A and the drug needle 16A are in parallel with and offset from the central axis C of the vial member 2A extending along the longitudinal direction L when the second rotating restraining structure 153A is non-rotatably engaged with the first rotating restraining structure 131A. However, the present invention is not limited to this embodiment. For example, in another embodiment, one of the air needle and the drug needle can be coincided with the central axis of the vial member.

Furthermore, as shown in FIG. 2, FIG. 3 and FIG. 4, the injection device 1A further includes a shielding member 18A and a resilient member 19A. The shielding member 18A is disposed on the injector body 11A and extendable or retractable relative to the injector body 11A along the longitudinal direction L to conceal or reveal the drug needle 16A, and the shielding member 18A is located inside a space defined between the vial adaptor 15A and the injector body 11A when the vial adaptor 15A is engaged with the needle hub 13A. The resilient member 19A is configured to drive the shielding member 18A to extend out of the injector body 11A along the longitudinal direction L to conceal the drug needle 16A for preventing a direct contact of the drug needle 16A when the cap 14A is removed from the injector body 11A along the longitudinal direction L. The shielding member 18A can be pressed by a patient's skin to retract relative to the injector body 11A and compress the resilient member 19A to reveal the drug needle 16A for allowing the drug needle 16A to penetrate the patient's skin.

Preferably, at least one guiding structure G is formed on the shielding member 18A and configured to cooperate with a portion of the injector body 11A for guiding a movement of the shielding member 18A. For example, the guiding structure G can be a guiding slot structure.

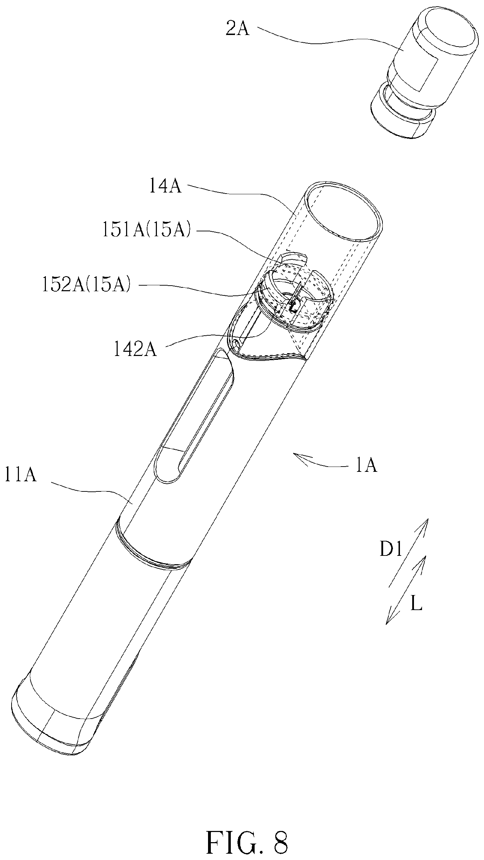

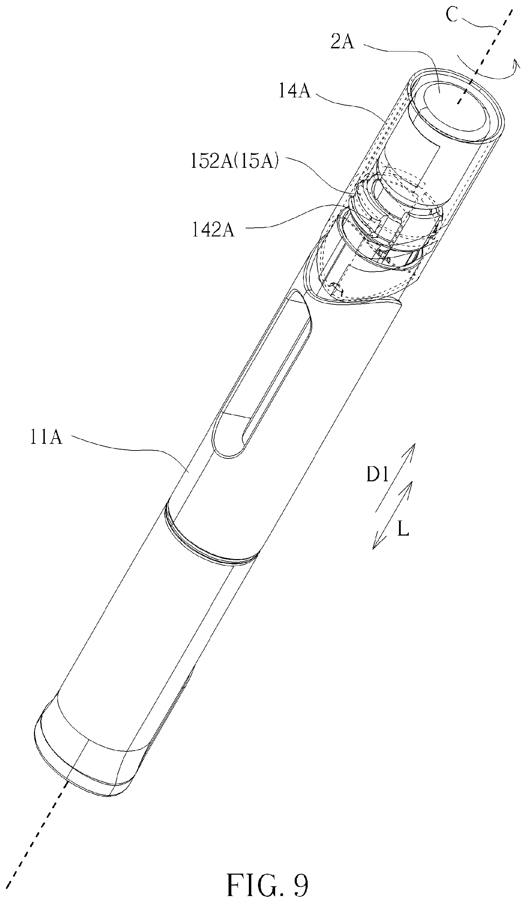

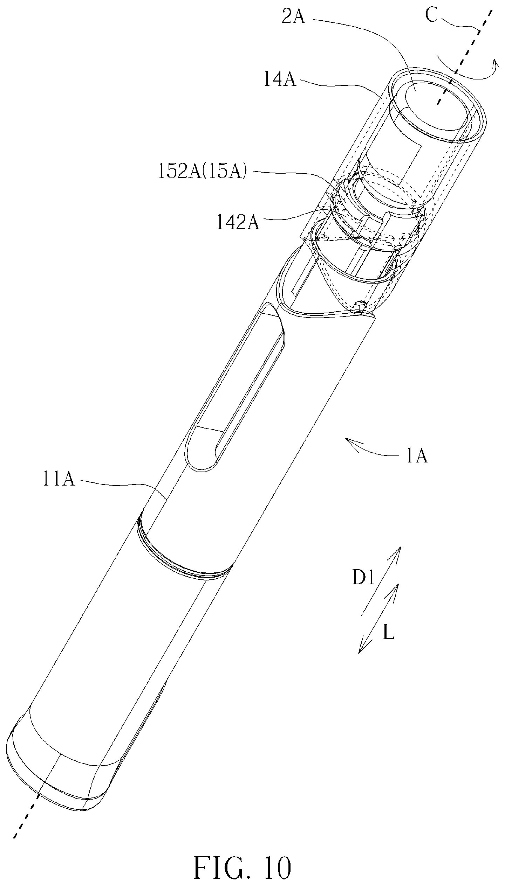

Please further refer to FIG. 4 and FIG. 8 to FIG. 10. FIG. 8 to FIG. 10 are partial diagrams of the injection device 1A in different states according to the first embodiment of the present invention. As shown in FIG. 4 and FIG. 8, the vial member 2A can be mounted on the injection device 1A and held by the resilient hook structure 151A of the vial adaptor 15A in an inserting manner through an opening of the cap 14A for allowing the drug needle 16A and the air needle 17A to penetrate the vial member 2A for the drug transfer. After completion of the drug transfer, the cap 14A can be rotated to move through a position as shown in FIG. 9 to a position as shown in FIG. 10 to drive the vial member 2A and the vial adaptor 15A to move along a first direction D1 longitudinally for removal of the vial member 2A. The injection device 1A allows the cap 14A to rotate without introducing rotation of the vial member 2A relative to the drug needle 16A and/or the air needle 17A, thereby effectively preventing the drug needle 16A and/or the air needle 17A from being deformed or damaged due to a torsional force during removal of the vial member 2A.

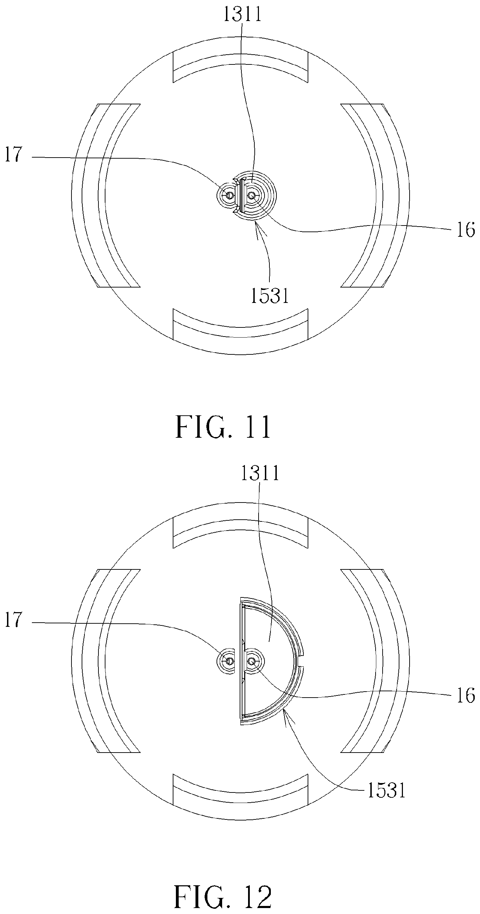

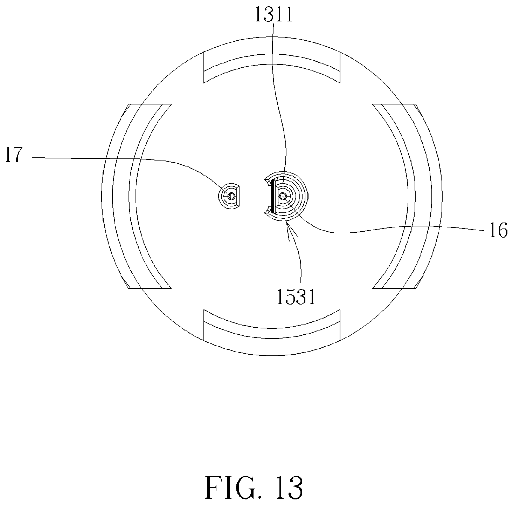

It should be noticed that the structural relationship between the vial adaptor 15A and the needle hub 13A is not limited to the one illustrated in the figures of this embodiment. For example, please refer to FIG. 11 to FIG. 13. FIG. 11 to FIG. 13 are partial diagrams of the injection device 1A according to different embodiments of the present invention. As shown in FIG. 10 to FIG. 13, a shape of a cross section of a restraining space 1531 and a shape of a cross section of a first restraining feature 1311 can vary according to practical demands, and a drug needle 16 and an air needle 17 can be located adjacent to or spaced apart from each other according to practical demands.

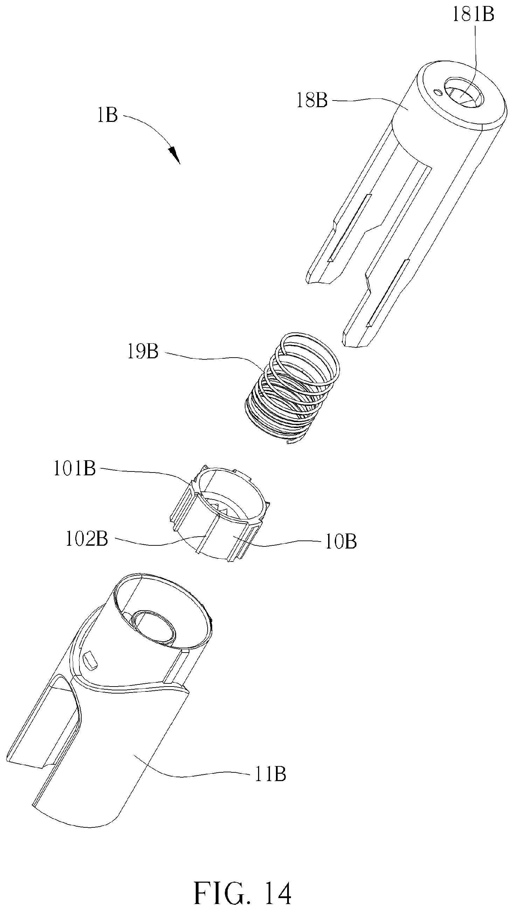

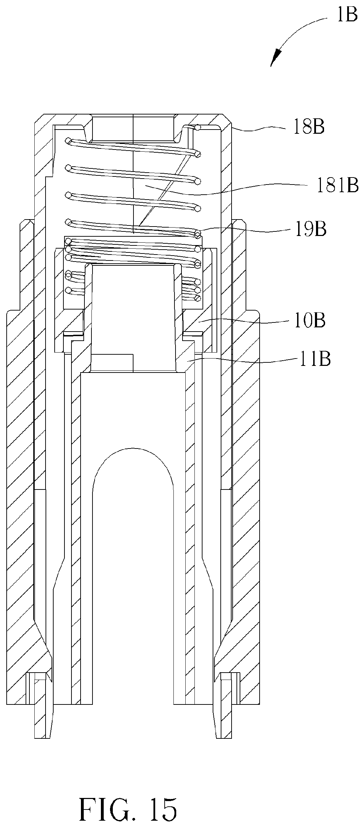

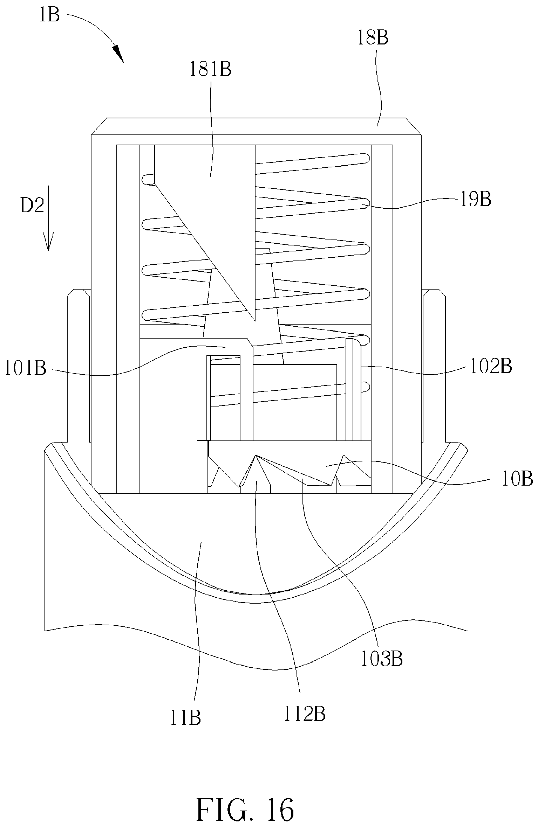

Please refer to FIG. 14 to FIG. 20. FIG. 14 is a partial diagram of an injection device 1B according to a second embodiment of the present invention. FIG. 15 is a partial exploded diagram of the injection device 1B according to the second embodiment of the present invention. FIG. 16 to FIG. 20 are partial diagrams of the injection device 1B in different states according to the second embodiment of the present invention. As shown in FIG. 14 to FIG. 20, the injection device 1B of the second embodiment is similar to the injection device 1A of the first embodiment. Different from the first embodiment, in this embodiment, the injection device 1B further includes a rotating member 10B disposed between an injector body 11B and a resilient member 19B. In other words, the resilient member 19B is abutted by and located between a shielding member 18B and the rotating member 10B. The shielding member 18B includes at least one driving structure 181B. The injector body 11B includes at least one first positioning structure 112B. The rotating member 10B includes at least one first cooperating structure 101B, at least one second cooperating structure 102B and at least one second positioning structure 103B.

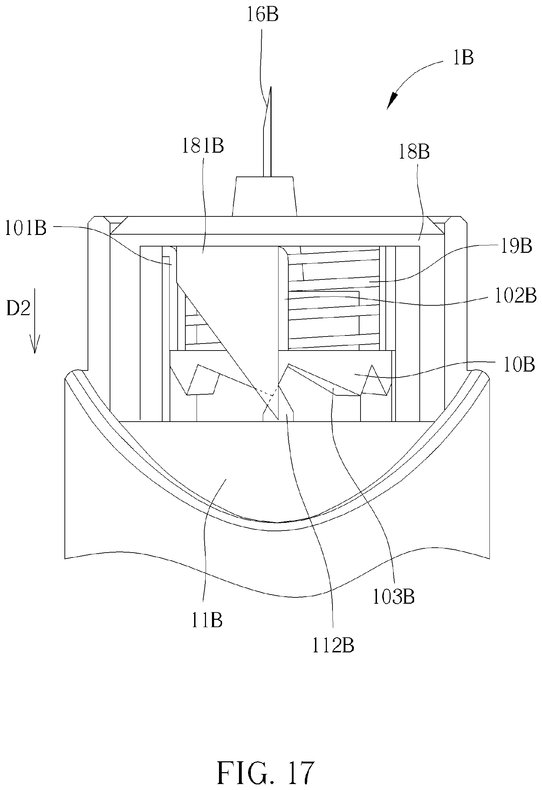

When the shielding member 18B is pressed from an extended position along a second direction D2 by the vial adaptor, which is not in the figures, during assembly of the injection device 1B, the driving structure 181B and the first cooperating structure 101B are configured to slidably abut against each other to drive the rotating member 10B to rotate from a position as shown in FIG. 16 to a position as shown in FIG. 17 to switch the injection device 1B from a state as shown in FIG. 16 to a state as shown in FIG. 17 to resiliently compress the resilient member 19B.

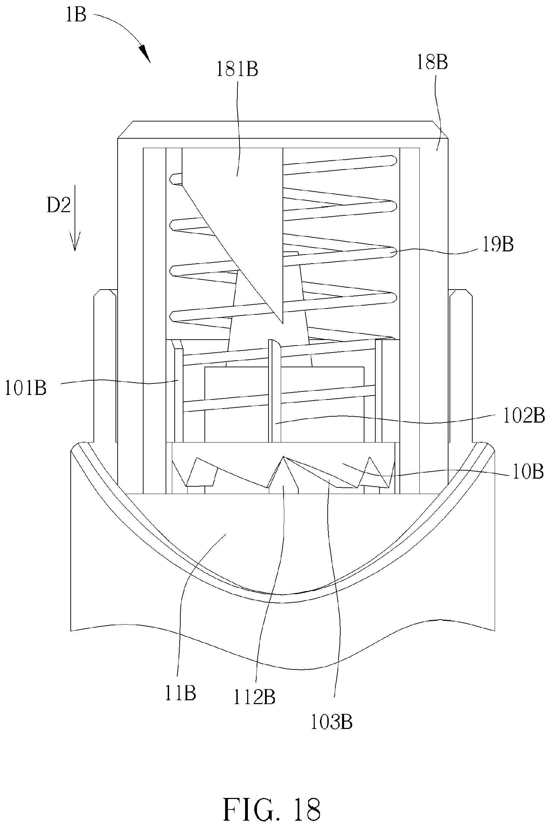

When the shielding member 18A is released by removal of the cap, which is not shown in the figures, due to resilient recovering of the resilient member 19B, the first positioning structure 112B and the second positioning structure 103B are configured to slidably abut against each other to drive the rotating member 10B to rotate from the position as shown in FIG. 17 to a position as shown in FIG. 18 to switch the injection device 1B from the state as shown in FIG. 17 to a state as shown in FIG. 18, and the shielding member 18B pops up back to the extended position.

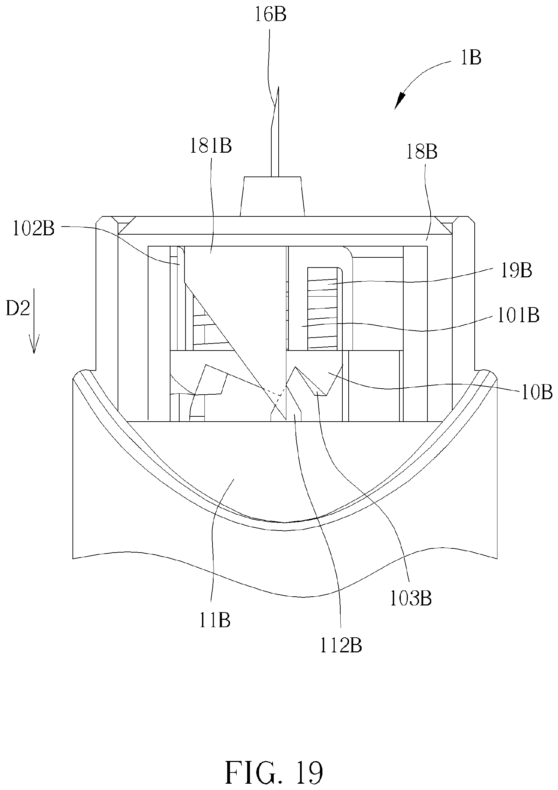

When the shielding member 18B is pressed from the extended position along the second direction D2 by a patient's skin during drug injection, the driving structure 181B and the second cooperating structure 102B are configured to slidably abut against each other to drive the rotating member 10B to rotate from the position as shown in FIG. 18 to a position as shown in FIG. 19 to switch the injection device 1B from the state as shown in FIG. 18 to a state as shown in FIG. 19 to resiliently compress the resilient member 19B.

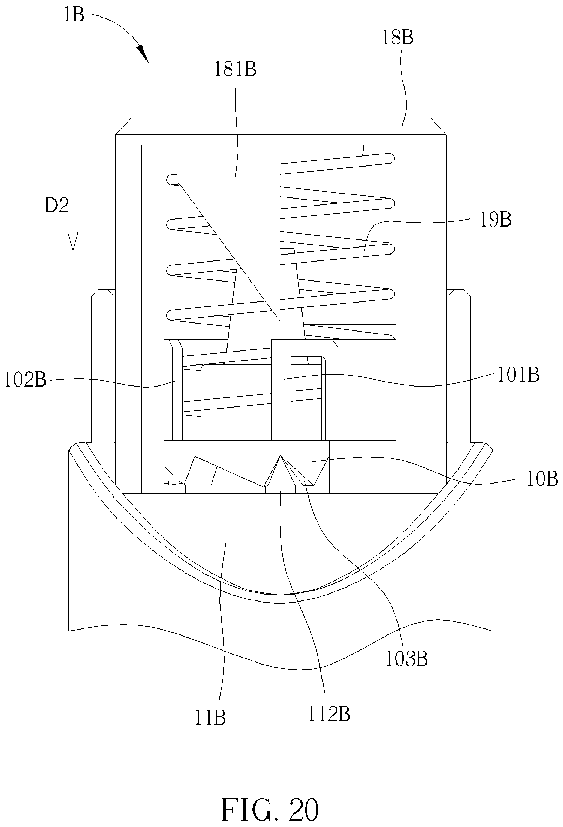

When the shielding member 18B is released after completion of the drug injection, due to resilient recovering of the resilient member 19B, the first positioning structure 112B and the second positioning structure 103B are configured to slidably abut against each other to drive the rotating member 10B to rotate from the position as shown in FIG. 19 to a position as shown in FIG. 20 to switch the injection device 1B from the state as shown in FIG. 19 to a state as shown in FIG. 20, and the shielding member 18B pops up back to the extended position.

In this embodiment, when the injection device 1B is in the state as shown in FIG. 20, the first cooperating structure 101B is configured to block the driving structure 181B for restraining the shielding member 18B from retracting along the second direction D2, thereby preventing exposure of a drug needle 16B after the drug injection.

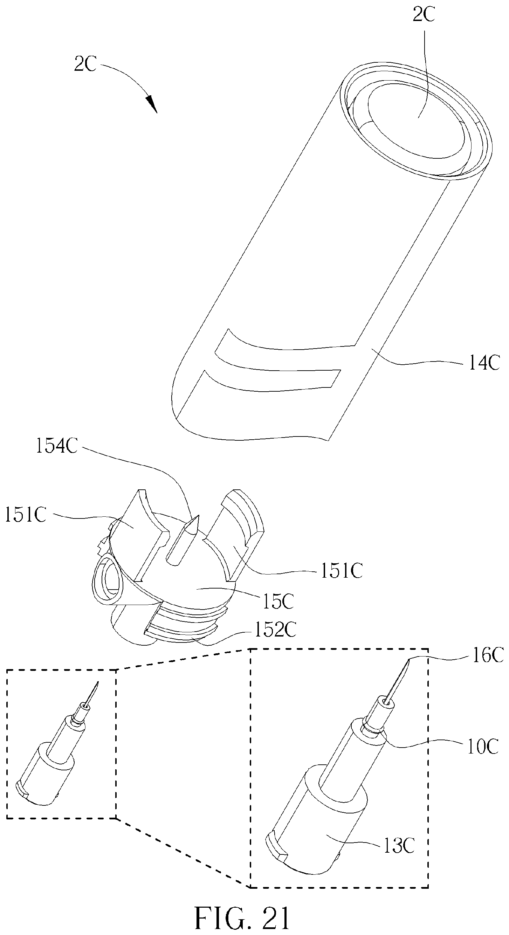

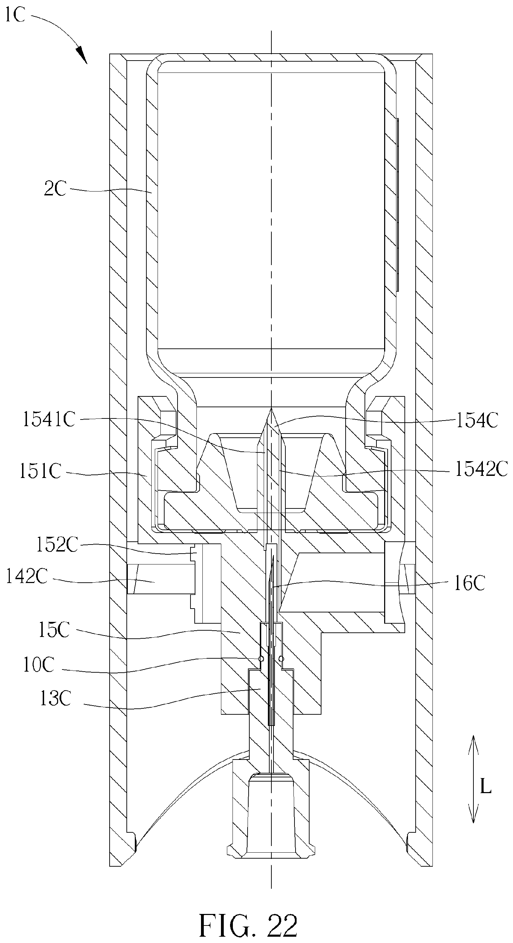

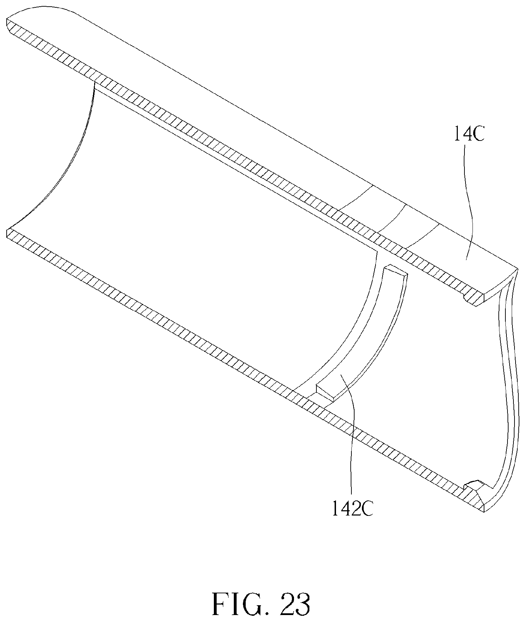

Please refer to FIG. 21 to FIG. 23. FIG. 21 is a partial exploded diagram of an injection device 1C according to a third embodiment of the present invention. FIG. 22 is a partial sectional diagram of the injection device 1C according to the third embodiment of the present invention. FIG. 23 is a partial diagram of a cap 14C according to the third embodiment of the present invention. As shown in FIG. 21 to FIG. 23, the injection device 1C of the third embodiment is similar to the injection device 1A of the first embodiment. Different from the first embodiment, in the third embodiment, a vial adaptor 15C includes a piercing structure 154C configured to pierce into a vial member 2C, and the piercing structure 154C is configured to provide a drug channel 1541C for allowing drug to flow out of the vial member 2C when the piercing structure 154C pierces into the vial member 2C. A drug needle 16C and the piercing structure 154C are in parallel with a central axis of the vial member 2C extending along the longitudinal direction L. Specifically, the drug needle 16C and the piercing structure 154C are coincided with the central axis of the vial member 2C. A head portion of a needle hub 13C is configured to at least partially stretch into the vial adaptor 15C, and the drug needle 16C is located inside the vial adaptor 15C and configured to provide a drug passage communicated with the drug channel 1541C when the head portion of the needle hub 13C at least partially stretches into the vial adaptor 15C. In this embodiment, since there is no torsional force exerting on the drug needle 16C directly, the head portion of the needle hub 13C can be engaged with the vial adaptor 15C in a rotatable or non-rotatable manner.

Besides, the injection device 1C further includes a sealing member 10C configured to be engaged between the vial adaptor 15C and the head portion of the needle hub 13C for preventing any drug leakage. For example, the sealing member 10C can be a rubber O-ring.

Preferably, the piercing structure 154C is further configured to provide an air channel 1542C for allowing air to flow into the vial member 2C when the piercing structure 154C pierces into the vial member 2C. In other words, in this embodiment, no air needle is provided on the vial adaptor 15C.

Furthermore, in this embodiment, a resilient hook structure 151C for hold the vial member 2C and a second rotating cooperating structure 152C for engaging with a first rotating cooperating structure 142C of the cap 14C are located at two opposite sides of the vial adaptor 15C, respectively.

Other details of this embodiment are the same as the ones of the first embodiment. Detailed description is omitted herein for simplicity.

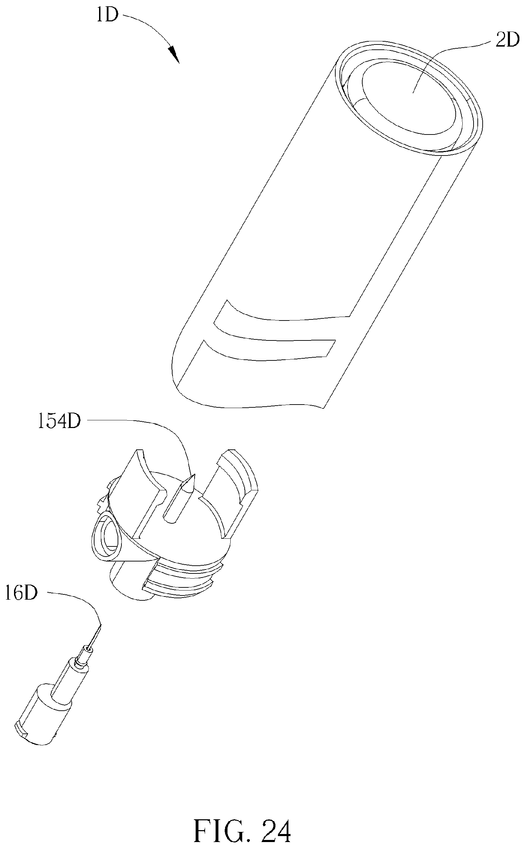

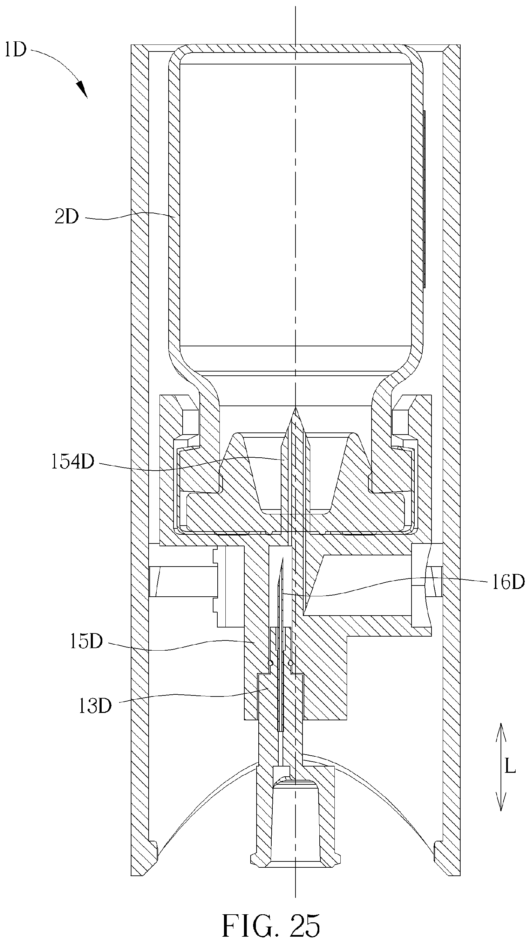

Please refer to FIG. 24 to FIG. 25. FIG. 24 is a partial exploded diagram of an injection device 1D according to a fourth embodiment of the present invention. FIG. 25 is a partial sectional diagram of the injection device 1D according to the fourth embodiment of the present invention. As shown in FIG. 24 to FIG. 25, the injection device 1D of the fourth embodiment is similar to the injection device 1C of the third embodiment. Different from the third embodiment, in the fourth embodiment, a piercing structure 154D is coincided with a central axis of a vial member 2D, and a drug needle 16D is offset from the central axis of the vial member 2D. Other details of this embodiment are the same as the ones of the third embodiment. Detailed description is omitted herein for simplicity.

In contrast to the prior art, the injection device of the present invention is suitable for vial-based drug preparation and capable of preventing the drug needle from being deformed or damaged due to a torsional force during removal of the vial member. As a result, the injection device of the present invention enhances reliability and operational convenience in drug administration.

Those skilled in the art will readily observe that numerous modifications and alterations of the device and method may be made while retaining the teachings of the invention. Accordingly, the above disclosure should be construed as limited only by the metes and bounds of the appended claims.

## Claims

1. An injection device (1A, 1B, 1C, 1D) adapted for a vial member (2A, 2C, 2D) having a central axis (C) of the vial member (2A) extending along a longitudinal direction (L), the injection device (1A, 1B, 1C, 1D) **characterized by**:
an injector body (11A, 11B);
a reservoir (12A) disposed on the injector body (11A, 11B);
a needle hub (13A, 13C) engaged with the reservoir (12A);
a cap (14A, 14C) removably mounted on the injector body (11A, 11B);
a vial adaptor (15A, 15C) separably engaged with the needle hub (13A, 13C), the vial adaptor (15A, 15C) being configured to hold the vial member (2A, 2C, 2D); and
a drug needle (16A, 16B, 16C, 16D, 16) disposed on the needle hub (13A, 13C) and configured to provide a drug passage communicated with an interior of the reservoir (12A) for allowing drug to flow from an interior of the vial member (2A, 2C, 2D) to the interior of the reservoir (12A).

2. The injection device (1A, 1B) of claim 1, **characterized in that** the needle hub (13A) comprises a first rotating restraining structure (131A), the vial adaptor (15A) comprises a second rotating restraining structure (153A) configured to non-rotatably engage with the first rotating restraining structure (131A) when the vial adaptor (15A) is engaged with the needle hub (13A), and a non-rotating engagement of the first rotating restraining structure (131A) and the second rotating restraining structure (153A) restrains a relative rotating movement of the vial adaptor (15A) and the needle hub (13A) around the central axis (C) of the vial member (2A) extending along the longitudinal direction (L) and allow a relative linear movement of the vial adaptor (15A) and the needle hub (13A) along the longitudinal direction (L).

3. The injection device (1A, 1B) of claim 2, **characterized in that** the first rotating restraining structure (131A) comprises a first restraining feature (1311A, 1311), the second rotating restraining structure (153A) comprises a restraining space (1531A, 1531) and a second restraining feature (1532A), and the first restraining feature (1311A, 1311) is configured to stretch into the restraining space (1531A, 1531) along the first restraining feature (1311A, 1311) for the non-rotating engagement of the first rotating restraining structure (131A) and the second rotating restraining structure (153A).

4. The injection device (1A, 1B) of any of claims 2 to 3, further **characterized by** an air needle (17A) disposed on or integrally formed with the second rotating restraining structure (153A) of the vial adaptor (15A) and configured to pierce into the vial member (2A) to provide an air passage communicated with the interior of the vial member (2A) for allowing air to flow to the interior of the vial member (2A), the drug needle (16A, 16B, 16) being disposed on the first rotating restraining structure (131A) of the needle hub (13A) and configured to pierce into the vial member (2A), and the air needle (17A) and the drug needle (16A, 16B, 16) being located adjacent to each other and in parallel with the central axis (C) of the vial member (2A) extending along the longitudinal direction (L) when the second rotating restraining structure (153A) is non-rotatably engaged with the first rotating restraining structure (131A).

5. The injection device (1A, 1B, 1C, 1D) of any of claims 1 to 4, further **characterized by** a shielding member (18A, 18B) disposed on the injector body (11A, 11B) and extendable or retractable relative to the injector body (11A, 11B) along the longitudinal direction (L) to conceal or reveal the drug needle (16A, 16B, 16C, 16D, 16), and the shielding member (18A, 18B) being located inside a space defined between the vial adaptor (15A, 15C) and the injector body (11A, 11B) when the vial adaptor (15A, 15C) is engaged with the needle hub (13A, 13C).

6. The injection device (1A, 1B, 1C, 1D) of claim 5, further **characterized by** a resilient member (19A, 19B) configured to drive the shielding member (18A, 18B) to extend out of the injector body (11A, 11B) along the longitudinal direction (L) to conceal the drug needle (16A, 16B, 16C, 16D, 16) when the cap (14A, 14C) is removed from the injector body (11A, 11B) along the longitudinal direction (L).

7. The injection device (1A, 1B, 1C, 1D) of any of claims 1 to 6, **characterized in that** the cap (14A, 14C) is configured to receive the vial adaptor (15A, 15C) and the vial member (2A, 2C, 2D), and the vial adaptor (15A, 15C) is configured to receive a portion of the vial member (2A, 2C, 2D).

8. The injection device (1A, 1B, 1C, 1D) of claim 7, **characterized in that** the vial adaptor (15A, 15C) comprises at least one resilient hook structure (151A) configured to hold the vial member (2A, 2C, 2D), the cap (14A, 14C) comprises a wall section (141A) configured to orientate the vial member (2A, 2C, 2D) for aligning a length direction of the vial member (2A, 2C, 2D) with the longitudinal direction (L), and the wall section (141A) encircles the vial member (2A, 2C, 2D) when the at least one resilient hook structure (151A) holds the vial member (2A, 2C, 2D).

9. The injection device (1A, 1B, 1C, 1D) of any of claims 1 to 8, **characterized in that** the cap (14A, 14C) comprises at least one first rotating cooperating structure (142A, 142C), the vial adaptor (15A, 15C) comprises at least one second rotating cooperating structure (152A, 152C) configured to rotatably engage with the at least one first rotating cooperating structure (142A, 142C), and when the cap (14A, 14C) rotates relative to the injector body (11A, 11B) around the central axis (C) of the vial member (2A, 2C, 2D) extending along the longitudinal direction (L), a rotating engagement of the at least one first rotating cooperating structure (142A, 142C) and the at least one second rotating cooperating structure (152A, 152C) enables the cap (14A, 14C) to rotate relative to the vial adaptor (15A, 15C).

10. The injection device (1A, 1B, 1C, 1D) of claim 9, **characterized in that** when the cap (14A, 14C) is removed from the injector body (11A, 11B) along the longitudinal direction (L), the rotating engagement of the at least one first rotating cooperating structure (142A, 142C) and the at least one second rotating cooperating structure (152A, 152C) enables the cap (14A, 14C) to drive the vial adaptor (15A, 15C) and the vial member (2A, 2C, 2D) to move together with the cap (14A, 14C) along the longitudinal direction (L).

11. The injection device (1A, 1B, 1C, 1D) of any of claims 9 to 10, **characterized in that** one of the at least one first rotating cooperating structure (142A, 142C) and the at least one second rotating cooperating structure (152A, 152C) is a single-rib structure, another one of the at least one first rotating cooperating structure (142A, 142C) and the at least one second rotating cooperating structure (152A, 152C) is a double-rib structure, and the single-rib structure is movably located between two rib portions of the double-rib structure.

12. The injection device (1A, 1B, 1C, 1D) of any of claims 9 to 11, **characterized in that** the injector body (11A, 11B) comprises a first abutting structure (111A), and the cap (14A, 14C) further comprises a second abutting structure (143A) configured to cooperate with the first abutting structure (111A) to drive the cap (14A, 14C) to move along the longitudinal direction (L) for removal of the cap (14A, 14C) from the injector body (11A, 11B) when the cap (14A, 14C) rotates relative to the injector body (11A, 11B).

13. The injection device (1C, 1D) of any of claims 1 and 5 to 12, **characterized in that** the vial adaptor (15C) comprises a piercing structure (154D) configured to pierce into the vial member (2C, 2D), the piercing structure (154D) is configured to provide a drug channel (1541C), the drug needle (16C, 16D) and the piercing structure (154C, 154D) are in parallel with the central axis (C) of the vial member (2C, 2D) extending along the longitudinal direction (L), and a head portion of the needle hub (13C) is configured to at least partially stretch into the vial adaptor (15C).

14. The injection device (1C, 1D) of claim 13, further **characterized by** a sealing member (10C) configured to be engaged between the vial adaptor (15C) and the head portion of the needle hub (13C).

15. The injection device (1C, 1D) of any of claims 13 to 14, **characterized in that** the piercing structure (154C, 154D) is further configured to provide an air channel (1542C) communicated with the interior of the vial member (2C, 2D) for allowing air to flow to the interior of the vial member (2C, 2D).

## Amended claims

### Amended claims in accordance with Rule 137(2) EPC.

1. An injection device (1A, 1B) adapted for a vial member (2A) having a central axis (C) of the vial member (2A) extending along a longitudinal direction (L), the injection device (1A, 1B) comprising:
an injector body (11A, 11B);
a reservoir (12A) disposed on the injector body (11A, 11B);
a needle hub (13A) engaged with the reservoir (12A);
a cap (14A) removably mounted on the injector body (11A, 11B);
a vial adaptor (15A) separably engaged with the needle hub (13A), the vial adaptor (15A) being configured to hold the vial member (2A); and
a drug needle (16A, 16B, 16) disposed on the needle hub (13A) and configured to provide a drug passage communicated with an interior of the reservoir (12A) for allowing drug to flow from an interior of the vial member (2A) to the interior of the reservoir (12A);
further **characterized by** an air needle (17A);
wherein the needle hub (13A) comprises a first rotating restraining structure (131A), the vial adaptor (15A) comprises a second rotating restraining structure (153A) configured to non-rotatably engage with the first rotating restraining structure (131A) when the vial adaptor (15A) is engaged with the needle hub (13A), and a non-rotating engagement of the first rotating restraining structure (131A) and the second rotating restraining structure (153A) restrains a relative rotating movement of the vial adaptor (15A) and the needle hub (13A) around the central axis (C) of the vial member (2A) extending along the longitudinal direction (L) and allow a relative linear movement of the vial adaptor (15A) and the needle hub (13A) along the longitudinal direction (L);
wherein the air needle (17A) is disposed on or integrally formed with the second rotating restraining structure (153A) of the vial adaptor (15A) and configured to pierce into the vial member (2A) to provide an air passage communicated with the interior of the vial member (2A) for allowing air to flow to the interior of the vial member (2A), the drug needle (16A, 16B, 16) is disposed on the first rotating restraining structure (131A) of the needle hub (13A) and configured to pierce into the vial member (2A), and the air needle (17A) and the drug needle (16A, 16B, 16) are located adjacent to each other and in parallel with the central axis (C) of the vial member (2A) extending along the longitudinal direction (L) when the second rotating restraining structure (153A) is non-rotatably engaged with the first rotating restraining structure (131A).

2. The injection device (1A, 1B) of claim 1, **characterized in that** the first rotating restraining structure (131A) comprises a first restraining feature (1311A, 1311), the second rotating restraining structure (153A) comprises a restraining space (1531A, 1531) and a second restraining feature (1532A), and the first restraining feature (1311A, 1311) is configured to stretch into the restraining space (1531A, 1531) along the first restraining feature (1311A, 1311) for the non-rotating engagement of the first rotating restraining structure (131A) and the second rotating restraining structure (153A).

3. The injection device (1A, 1B) of any of claims 1 to 2, further **characterized by** a shielding member (18A, 18B) disposed on the injector body (11A, 11B) and extendable or retractable relative to the injector body (11A, 11B) along the longitudinal direction (L) to conceal or reveal the drug needle (16A, 16B, 16), and the shielding member (18A, 18B) being located inside a space defined between the vial adaptor (15A) and the injector body (11A, 11B) when the vial adaptor (15A) is engaged with the needle hub (13A).

4. The injection device (1A, 1B) of claim 3, further **characterized by** a resilient member (19A, 19B) configured to drive the shielding member (18A, 18B) to extend out of the injector body (11A, 11B) along the longitudinal direction (L) to conceal the drug needle (16A, 16B, 16) when the cap (14A) is removed from the injector body (11A, 11B) along the longitudinal direction (L).

5. The injection device (1A, 1B) of any of claims 1 to 4, **characterized in that** the cap (14A) is configured to receive the vial adaptor (15A) and the vial member (2A), and the vial adaptor (15A) is configured to receive a portion of the vial member (2A).

6. The injection device (1A, 1B) of claim 5, **characterized in that** the vial adaptor (15A) comprises at least one resilient hook structure (151A) configured to hold the vial member (2A), the cap (14A) comprises a wall section (141A) configured to orientate the vial member (2A) for aligning a length direction of the vial member (2A) with the longitudinal direction (L), and the wall section (141A) encircles the vial member (2A) when the at least one resilient hook structure (151A) holds the vial member (2A).

7. The injection device (1A, 1B) of any of claims 1 to 6, **characterized in that** the cap (14A) comprises at least one first rotating cooperating structure (142A), the vial adaptor (15A) comprises at least one second rotating cooperating structure (152A) configured to rotatably engage with the at least one first rotating cooperating structure (142A), and when the cap (14A) rotates relative to the injector body (11A, 11B) around the central axis (C) of the vial member (2A) extending along the longitudinal direction (L), a rotating engagement of the at least one first rotating cooperating structure (142A) and the at least one second rotating cooperating structure (152A) enables the cap (14A) to rotate relative to the vial adaptor (15A).

8. The injection device (1A, 1B) of claim 7, **characterized in that** when the cap (14A) is removed from the injector body (11A, 11B) along the longitudinal direction (L), the rotating engagement of the at least one first rotating cooperating structure (142A) and the at least one second rotating cooperating structure (152A) enables the cap (14A) to drive the vial adaptor (15A) and the vial member (2A) to move together with the cap (14A) along the longitudinal direction (L).

9. The injection device (1A, 1B) of any of claims 7 to 8, **characterized in that** one of the at least one first rotating cooperating structure (142A) and the at least one second rotating cooperating structure (152A) is a single-rib structure, another one of the at least one first rotating cooperating structure (142A) and the at least one second rotating cooperating structure (152A) is a double-rib structure, and the single-rib structure is movably located between two rib portions of the double-rib structure.

10. The injection device (1A, 1B) of any of claims 7 to 9, **characterized in that** the injector body (11A, 11B) comprises a first abutting structure (111A), and the cap (14A) further comprises a second abutting structure (143A) configured to cooperate with the first abutting structure (111A) to drive the cap (14A) to move along the longitudinal direction (L) for removal of the cap (14A) from the injector body (11A, 11B) when the cap (14A) rotates relative to the injector body (11A, 11B).
